# EUROPEAN PATENT APPLICATION

(11) **EP 3 263 024 A1**
(43) Date of publication of application: **03.01.2018**
(21) Application number: 16198048.7
(22) Date of filing: 09.11.2016
(51) Int. Cl.: A61B 5/024, A61B 5/16, A61B 5/00, A61B 5/0402

(54) **EMOTION ANALYSIS METHOD AND ELECTRONIC APPARATUS THEREOF**

(30) Priority: 30.06.2016 TW 105120706
(71) Applicant: Cal-Comp Electronics & Communications Company Limited, New Taipei City 22201 (TW); Kinpo Electronics, Inc., New Taipei City 22201 (TW)
(72) Inventor: GUO, Han-Wen, New Taipei City 22201 (TW); CHIEN, Jen-Chien, New Taipei City 22201 (TW)
(74) Representative: Chamberlain, Alan James

(57) **Abstract**

An emotion analysis method and an electronic apparatus thereof are provided. The emotion analysis method is adapted to the electronic apparatus having a database or connected to the database in order to analyze an emotion of an examinee. The emotion analysis method includes: obtaining a heart rate signal of the examinee; defining a plurality of candidate emotions from the database; analyzing the heart rate signal to obtain a plurality of target emotion parameters; and analyzing the target emotion parameters to determine one of the candidate emotions corresponding to the heart rate signal by applying an emotion analysis model.

## Description

### Technical Field

The present disclosure relates to an analysis method and an apparatus thereof, and more particularly, to an emotion analysis method and an electronic apparatus thereof, which are adapted to analyze heart rate signals.

### BACKGROUND

In recent years, there are increasingly more researches indicating that emotions are in close relationship to human health. The researchers found that, in the blood of a person who stayed under chronic repression for years may have increasing glucose and fatty acid which lead to higher risks of suffering diabetes and heart disease. Also, pressure may also increase the level of cholesterol in human body to induce cardiovascular disease more easily. Therefore, maintaining a positive and optimistic emotion may be a way to improve body resistance and enable people to live longer; conversely, the person with emotion stayed under depression, frustration or anger for years may have dysfunction of the immune system to thereby causing various diseases. Further, emotional dissonance may further induce social withdrawal and even lower work efficiency.

Therefore, an emotion analysis becomes an important factor for determining physical and mental state of one person. Today, a subjective emotion determination is mainly achieved by a questionnaire, whereas the most used method for an objective emotion determination is achieved through physiological features such as a facial expression or a heart rate analysis. Nonetheless, it is very difficult to objectively determine a true emotion of the examinee regardless of whether the determination adopts use of the questionnaire or the facial expression. Hence, the heart rate analysis is a more objective and systemic approach for determining the emotion of the examinee nowadays. However, because the existing heart rate analysis methods are mainly focused on single heart rate variation such as increases and decreases in the heart rate, there is a higher probability that false positives may occur. Accordingly, it is still one of the major subjects for person skilled in the art as how to provide a more accurate and detailed heart rate analysis method.

### SUMMARY

Based on the above, the present disclosure proposes an emotion analysis method and an electronic apparatus thereof. After a plurality of different target emotion parameters are obtained by analyzing a heart rate signal through various statistical analysis methods, a comprehensive analysis is then performed on the target emotion parameters correspondingly. With the comprehensive analysis performed on the target emotion parameters obtained from the heart rate signal, an emotion of an examinee may be determined more objectively and accurately.

The present disclosure provides an emotion analysis method adapted for an emotion analysis system, which is adapted to an electronic apparatus having a database or connecting to the database in order to analyze an emotion of an examinee. The emotion analysis method includes the following steps. A heart rate signal of the examinee is obtained. A plurality of candidate emotions from the database is defined. The heart rate signal is analyzed to obtain a plurality of target emotion parameters. The target emotion parameters are analyzed to determine one of the candidate emotions corresponding to the heart rate signal by applying an emotion analysis model.

The present disclosure provides an electronic apparatus for analyzing an emotion of an examinee. The electronic apparatus includes an information extraction device and a processor. The information extraction device obtains an electrocardiogram signal from the examinee. The processor is coupled to the information extraction device. The processor obtains a heart rate signal of the examinee from the electrocardiogram signal and defines a plurality of candidate emotions from a database. The processor analyzes the heart rate signal to obtain a plurality of target emotion parameters, and analyzes the target emotion parameters to determine one of the candidate emotions corresponding to the heart rate signal by applying an emotion analysis model.

In view of the above, with use of the emotion analysis method and the electronic apparatus thereof as described above, an emotion state of the examinee may be analyzed more objectively and accurately when the target emotion parameters in the heart rate signal may be comprehensively analyzed.

To make the above features and advantages of the present disclosure more comprehensible, several embodiments accompanied with drawings are described in detail as follows.

### BRIEF DESCRIPTION OF THE DRAWINGS

The accompanying drawings are included to provide a further understanding of the present disclosure, and are incorporated in and constitute a part of this specification. The drawings illustrate embodiments of the present disclosure and, together with the description, serve to explain the principles of the present disclosure.
FIG. 1 is a block diagram of an electronic apparatus in an embodiment of the present disclosure.
FIG. 2 is a block diagram of an emotion analysis system in an embodiment of the present disclosure.
FIG. 3 is a block diagram of an emotion analysis system in another embodiment of the present disclosure.
FIG. 4 is a flowchart of an emotion analysis method in an embodiment of the present disclosure.
FIG. 5 is a schematic diagram of the electrocardiogram signal in an embodiment of the present disclosure.

### DETAILED DESCRIPTION

In the following detailed description, for purposes of explanation, numerous specific details are set forth in order to provide a thorough understanding of the disclosed embodiments. It will be apparent, however, that one or more embodiments may be practiced without these specific details. In other instances, well-known structures and devices are schematically shown in order to simplify the drawing.

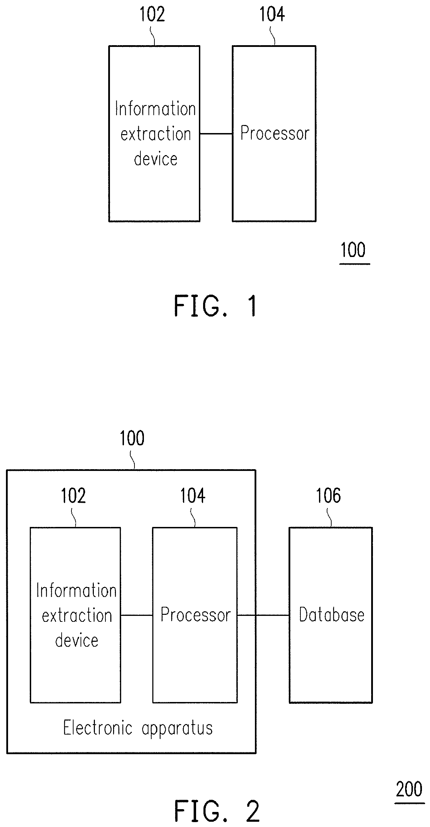

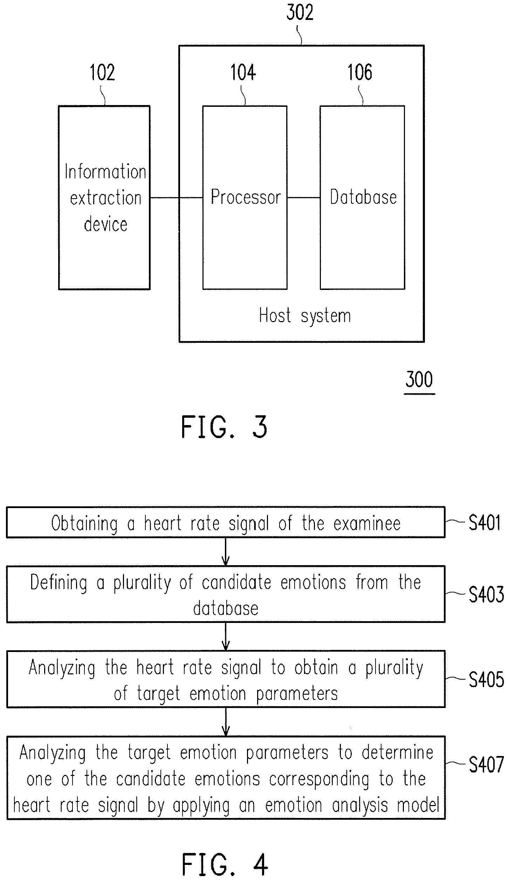

FIG. 1 is a block diagram of an electronic apparatus 100 in an embodiment of the present disclosure. FIG. 2 is a block diagram of an emotion analysis system 200 in an embodiment of the present disclosure. FIG. 3 is a block diagram of an emotion analysis system 300 in another embodiment of the present disclosure.

Referring to FIG. 1, the electronic apparatus 100 includes an information extraction device 102 and a processor 104. In an embodiment of the present disclosure, the electronic apparatus 100 obtains a heart rate signal of an examinee and conducts various analyses on the obtained heart rate signal, so as to determine the emotion state of the examinee. The information extraction device 102 is configured to serve as a medium for obtaining the heart rate signal of the examinee. In an embodiment of the present disclosure, the information extraction device 102 measures the electrocardiogram signal of the examinee, for example. In that case, the information extraction device 102 may be an electrocardiogram machine or a portable electrocardiography monitor, configured to obtain the electrocardiogram signal of the examinee. The processor 104 is coupled to the information extraction device 102, and configured to receive the electrocardiogram signal from the information extraction device 102, obtain the heart rate signal from the electrocardiogram signal, analyze the obtained heart rate signal, and determine the emotion state of the examinee or the emotion state reflected by the electrocardiogram signal according to an analysis result. In an embodiment of the present disclosure, the processor 104 may be, for example, a micro-controller, an embedded controller, a central processing unit (CPU) or similar elements, but the present disclosure is not limited to the above.

It is noted that, the electronic apparatus 100 in the embodiment of the present disclosure further includes, example, a storage unit (not illustrated), a communication interface (not illustrated) and an input/output interface (not illustrated). The storage unit is configured to store data, information, module, application, and may be in form of a random access memory (RAM), a read-only memory (ROM), a flash memory or similar elements, or a combination of the aforementioned elements. The input/output interface includes elements for outputting or inputting messages and data, such as a display, a speaker, a keyboard, a mouse, a touch panel. The communication interface supports various wired communication standards and wireless communication standards so that the electronic apparatus 100 can communicate with other devices.

It is noted that, the electronic apparatus 100 in the embodiment of the present disclosure can be connected to a database 106 externally or via a network to form the emotion analysis system 200, where the database 106 is configured to store various data. However, the present disclosure is not limited thereto. In another embodiment of the present disclosure, the database 106 may be included in the electronic apparatus 100 and coupled to the processor 104. In yet another embodiment of the present disclosure, the emotion analysis system 300 includes the information extraction device 102, the processor 104 and the database 106. However, the processor 104 and the database 106 are included in one host system 302, whereas the information extraction device 102 is independently implemented in a different device or element.

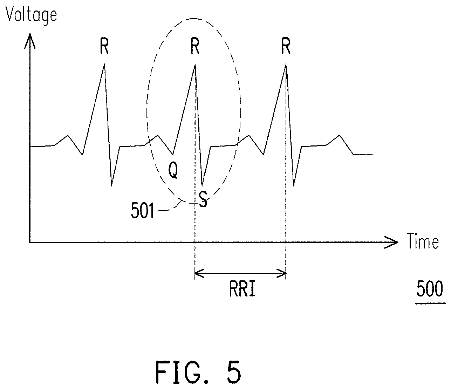

FIG. 4 is a flowchart of an emotion analysis method in an embodiment of the present disclosure. FIG. 5 is a schematic diagram of the electrocardiogram signal in an embodiment of the present disclosure.

Referring to FIG. 4, in step S401, the processor 104 obtains a heart rate signal to serve as an analysis target. In an embodiment of the present disclosure, the information extraction device 102 is, for example, the portable electrocardiography monitor configured to detect the electrocardiogram signal within a time interval (e.g., 90 seconds) and transmits the same back to the processor 104 in order to analyze the heart rate signal. The schematic diagram of FIG. 5 merely captures and illustrates 3 seconds among 90 seconds of the electrocardiogram signal for exemplary descriptions. In another embodiment of the present disclosure, the processor 104 may also obtain the heart rate signal to serve as the analysis target by using, for example, the database 106 or other methods. In step S403, the processor 104 further defines a plurality of candidate emotions from the database 106. Then, in step S405, the processor 104 analyzes and calculates the heart rate signal to obtain a plurality of target emotion parameters.

Referring to FIG. 5, generally, a horizontal axis represents time and a vertical axis represents voltage in an electrocardiogram signal 500. Usually, the electrocardiogram signal within 90 seconds includes a plurality of QRS wave groups 501. Among them, a peak of a first forward voltage appeared following a reverse voltage is marked as R, and a time interval between each adjacent two peaks R in the electrocardiogram signal 500 is known as a RR-interval (RRI) which represents a heartbeat cycle. In an embodiment of the present disclosure, the processor 104 obtains the electrocardiogram signal 500 within 90 seconds from the information extraction device 102, analyzes the electrocardiogram signal 500 to obtain the heart rate signal with the QRS wave group 501, and calculates a plurality of parameters of the heart rate signal in a time domain, a frequency domain, a statistical analysis and a Poincare plot to serve as a plurality of initial emotion parameters.

Specifically, the initial emotion parameters in the time domain include an average value of a plurality of RR-intervals of the heart rate signal, a coefficient of variation of the RR-intervals of the heart rate signal, a standard deviation of the RR-intervals of the heart rate signal, and a standard deviation of successive differences of the RR-intervals of the heart rate signal. In particular, the processor 104 can obtain the initial emotion parameters in the time domain by directly performing calculation on the electrocardiogram signal (e.g., the electrocardiogram signal 500) and the heart rate signal obtained in the time domain.

In an embodiment of the present disclosure, the processor 104 further transforms the obtained heart rate signal into the frequency domain for analysis in order to obtain a plurality of initial emotion parameters. The processor 104 can transform the heart rate signal into the frequency domain by a fast Fourier transform (FFT) and calculate powers of the heart rate signal respectively in a low frequency interval (0.04Hz to 0.15Hz) and a high frequency interval (0.15Hz to 0.4Hz) in order to obtain a low frequency (LF) power and a high frequency (HF) power of the heart rate signal to serve as the initial emotion parameters. On the other hand, the processor 104 can further calculate a ratio of the LF power and the HF power of the heart rate signal to serve as the initial emotion parameter.

It should be noted that, in another embodiment of the present disclosure, the processor 104 can also transform the heart rate signal into the frequency domain by other methods such as a Fourier transform or a Laplace transform, which are not particularly limited by the present disclosure.

In an embodiment of the present disclosure, the processor 104 can also calculate a plurality of initial emotion parameters by using, for example, a statistical analysis. More specifically, the processor 104 calculates a kurtosis and a skewness of the RR-intervals of the heart rate signal to serve as a plurality of initial emotion parameters.

In an embodiment of the present disclosure, the processor 104 further uses a standard deviation SD1 (a first standard deviation) and a standard deviation SD2 (a second standard deviation) of the heart rate signal in the Poincare plot and a ratio of the standard deviation SD2 and the standard deviation SD1 to serve as the initial emotion parameters. The Poincare plot has the advantage of simplicity in calculation and is suitable for data analysis in short period of time. The standard deviation SD1 is a standard deviation among data points perpendicular to a line of identity, whereas the standard deviation SD2 is a standard deviation among data points along the line of identity. It should be noted that, other characteristics or statistic values in the heart rate signal may also serve as the initial emotion parameters, which are not limited only to the above.

After obtaining the initial emotion parameters, the processor 104 selects at least part of the initial emotion parameters to serve the target emotion parameters. In an embodiment of the present disclosure, the processor 104, for example, uses all the initial emotion parameters to serve as the target emotion parameters for the subsequent emotion analysis, but the present disclosure is not limited thereto. In another embodiment, the processor 104 selects the part of the initial emotion parameters to serve as the target emotion parameters by performing a principal components analysis (PCA).

In the principal components analysis, the processor 104 compares a plurality of characteristic vectors of the initial emotion parameters with characteristic values of the heart rate signal, so as to select the part of the initial emotion parameters to serve as the target emotion parameters. Specifically, in the present embodiment, the initial emotion parameters selected by using the PCA are usually emotion parameters that can easily cause data variation. In an embodiment of the present disclosure, the selected initial emotion parameters are, for example, the coefficient of variation of the RR-intervals of the heart rate signal, the low frequency (LF) power of the heart rate signal, the high frequency (HF) power of the heart rate signal, the ratio of the LF power and the HF power and the standard deviation SD1 (the first standard deviation) of the heart rate signal in the Poincare plot, but the present disclosure is not limited to the above.

Referring to FIG. 4, after obtaining the target emotion parameters, the processor 104 further analyzes the target emotion parameters to determine one of the candidate emotions corresponding to the heart rate signal by applying an emotion analysis model (step S407). In general, variation on one or more target emotion parameters may correspond to variation on a certain type of emotion. Therefore, in the embodiment of the present disclosure, the processor 104 can determine the type of emotion corresponding to the heart rate signal by a combination of values from the multiple target emotion parameters. In other words, the most possible type of emotion that the examinee is currently in may be determined by using the heart rate signal.

In an embodiment of the present disclosure, the processor 104 applies one trained emotion analysis model in order to assist determining the corresponding one of the candidate emotions. More specifically, after the candidate emotions are determined and before the candidate emotion corresponding to the heart rate signal is officially recognized, the processor 104 first obtains a plurality of training heart rate signals in correspondence to the determined candidate emotions, respectively, obtains a plurality of training emotion parameters from the training heart rate signals, respectively, and obtains the emotion analysis model by training a classifier according to the training emotion parameters. In the present embodiment, the training heart rate signals may be, for example, heart rate signals previously measured and stored in the storage unit of the electronic apparatus 100, or heart rate signals previously measured and stored in the database 106.

Specifically, each of the training heart rate signals particularly corresponds to one type of the candidate emotions, such as sadness, anger, fear, joy or calm relaxation. In other words, the training heart rate signal is a heart rate signal of the examinee in a particular emotion. On the other hand, the training emotion parameters of the training heart rate signals are identical the types of the initial emotion parameters mentioned in the foregoing embodiments, which are not repeated hereinafter. With use of the training emotion parameters of the training heart rate signals, the processor 104 trains the classifier by using a support vector machine (SVM) in order to obtain the emotion analysis model, for example. Then, with use of the emotion analysis model, the processor 104 can then determine which candidate emotion each obtained heart rate signal is corresponding to.

In an embodiment of the present disclosure, those more specific candidate emotions (the candidate emotions such as sadness, anger, fear, joy or calm relaxation) may also be roughly analyzed and grouped into a positive emotion and a negative emotion. The positive emotion includes joy, calm relaxation and the like, whereas the negative emotion includes sadness, anger, fear and the like. In this case, with use of the emotion analysis model, the processor 104 can then determine whether each obtained heart rate signal is corresponding to the positive emotion or the negative emotion, so as to further determine whether the examinee is in the positive emotion or the negative emotion. In other words, in the present embodiment, the training emotion parameters for training the emotion analysis model are only classified into either the positive emotion or the negative emotion, and whether the examinee is in the positive emotion or the negative emotion is determined by the obtained heart rate signal by applying the emotion analysis model.

In summary, according to the emotion analysis method and the electronic apparatus thereof as proposed by the embodiments of the present disclosure, a plurality of target emotion parameters may be obtained by analyzing the heart rate signal, and the most possible type of emotion corresponding to the heart rate signal may be determined by applying the emotion analysis method based on the selected candidate emotions. As a result, in comparison with the traditional emotion analysis method and types, more different target emotion parameters may be comprehensive assessed to analyze different emotions in more details.

## Claims

1. An emotion analysis method, adapted to an electronic apparatus (100) having a database (106) or connecting to the database (106) in order to analyze an emotion of an examinee, the emotion analysis method comprising:
obtaining a heart rate signal of the examinee (S401);
defining a plurality of candidate emotions from the database (S403);
analyzing the heart rate signal to obtain a plurality of target emotion parameters (S405); and
analyzing the target emotion parameters to determine one of the candidate emotions corresponding to the heart rate signal by applying an emotion analysis model (S407).

2. The emotion analysis method according to claim 1, wherein the step of analyzing the heart rate signal to obtain the target emotion parameters (S405) comprises:
calculating a plurality of parameters of the heart rate signal in a time domain, a frequency domain, a statistical analysis and a Poincare plot to serve as a plurality of initial emotion parameters; and
selecting at least part of the initial emotion parameters to serve as the target emotion parameters.

3. The emotion analysis method according to claim 2, wherein the step of selecting the at least part of the initial emotion parameters to serve as the target emotion parameters comprises:
selecting the at least part of the initial emotion parameters to serve as the target emotion parameters by performing a principal components analysis (PCA).

4. The emotion analysis method according to claim 2, wherein the initial emotion parameters comprises an average value of a plurality of RR-intervals (RRI) of the heart rate signal, a coefficient of variation of the RR-intervals (RRI) of the heart rate signal, a standard deviation of the RR-intervals (RRI) of the heart rate signal, a standard deviation of successive differences of the RR-intervals (RRI) of the heart rate signal, a low frequency (LF) power of the heart rate signal, a high frequency (HF) power of the heart rate signal, a ratio of the LF power and the HF power of the heart rate signal, a kurtosis of the heart rate signal, a skewness of the heart rate signal, a first standard deviation of the heart rate signal in the Poincare plot, a second standard deviation of the heart rate signal in the Poincare plot and a ratio of the second standard deviation and the first standard deviation of the heart rate signal in the Poincare plot.

5. The emotion analysis method according to claim 1, wherein before analyzing the target emotion parameters to determine the one of the candidate emotions corresponding to the heart rate signal by applying the emotion analysis model (S407), the emotion analysis method further comprises:
obtaining a plurality of training heart rate signals in correspondence to the candidate emotions, respectively;
obtaining a plurality of training emotion parameters from the training heart rate signals, respectively; and
obtaining the emotion analysis model by training a classifier according to the training emotion parameters.

6. An electronic apparatus (100), for analyzing an emotion of an examinee, the electronic apparatus (100) comprising:
an information extraction device (102), obtaining an electrocardiogram signal (500) from the examinee; and
a processor (104), coupled to the information extraction device (102),
wherein the processor (104) obtains a heart rate signal of the examinee from the electrocardiogram signal (500) and defines a plurality of candidate emotions from a database (106),
wherein the processor (104) analyzes the heart rate signal to obtain a plurality of target emotion parameters, and analyzes the target emotion parameters to determine one of the candidate emotions corresponding to the heart rate signal by applying an emotion analysis model.

7. The electronic apparatus (100) according to claim 6, wherein the processor (104) calculates a plurality of parameters of the heart rate signal in a time domain, a frequency domain, a statistical analysis and a Poincare plot to serve as a plurality of initial emotion parameters, and selects at least part of the initial emotion parameters to serve as the target emotion parameters.

8. The electronic apparatus (100) according to claim 7, wherein the processor (104) selects the at least part of the initial emotion parameters to serve as the target emotion parameters by performing a principal components analysis (PCA).

9. The electronic apparatus (100) according to claim 7, wherein the initial emotion parameters comprises an average value of a plurality of RR-intervals (RRI) of the heart rate signal, a coefficient of variation of the RR-intervals (RRI) of the heart rate signal, a standard deviation of the RR-intervals (RRI) of the heart rate signal, a standard deviation of successive differences of the RR-intervals (RRI) of the heart rate signal, a low frequency (LF) power of the heart rate signal, a high frequency (HF) power of the heart rate signal, a ratio of the LF power and the HF power of the heart rate signal, a kurtosis of the heart rate signal, a skewness of the heart rate signal, a first standard deviation of the heart rate signal in the Poincare plot, a second standard deviation of the heart rate signal in the Poincare plot and a ratio of the second standard deviation and the first standard deviation of the heart rate signal in the Poincare plot.

10. The electronic apparatus (100) according to claim 6, wherein before analyzing the target emotion parameters to determine the one of the candidate emotions corresponding to the heart rate signal by applying the emotion analysis model, the processor (104) obtains a plurality of training heart rate signals in correspondence to the candidate emotions, respectively, obtains a plurality of training emotion parameters from the training heart rate signals, respectively, and obtains the emotion analysis model by training a classifier according to the training emotion parameters.
